# EUROPEAN PATENT SPECIFICATION

(11) **EP 4 461 251 B1**
(45) Date of publication and mention of the grant of the patent: **04.11.2026**
(21) Application number: 22920318.7
(22) Date of filing: 17.01.2022
(51) Int. Cl.: A61B 34/30, A61B 17/29

(54) **FORCEPS DEVICE**
ZANGENVORRICHTUNG
DISPOSITIF DE PINCE

(43) Date of publication of application: 13.11.2024
(73) Proprietor: RIVERFIELD Inc., 1070052 Tokyo (JP)
(72) Inventor: TAKIKAWA, Kyohei, Tokyo 160-0017 (JP)
(74) Representative: Adares PartGmbB
(86) International application number: PCT/JP2022/001340
(87) International publication number: WO 2023/135793

(56) References cited:
- JP-A- 2018 187 029
- JP-A- 2020 073 013
- JP-B1- 6 943 522
- KR-B1- 102 308 214
- US-A1- 2018 311 000
- US-A1- 2019 142 531
- US-A1- 2020 069 382
- US-A1- 2022 175 408
- US-A1- 2023 310 106

## Description

### Technical Field

The present invention relates to a forceps device used for a manipulator of a surgical robot.

### Background Art

Medical treatments using robots (manipulators) have recently been proposed in order to reduce the burden on operators and save manpower in medical facilities. In the field of surgery, proposals have been made for surgical manipulator systems for operators to treat patients by operating remotely-controllable surgical manipulators.

Various surgical tools are attached to leading ends of surgical manipulators. Known surgical tools include forceps for grasping human tissue during surgery. For example, Patent Literature 1 teaches a forceps device including a first rotating shaft (118) that turnably supports a support (107) holding a grasping part (101, 104), a base member (120) that holds the first rotating shaft, a plurality of first guide pulleys (111, 112, 113, 114) arranged coaxially with the first rotating shaft, a plurality of grasping-portion wires (102, 103, 105, 106) that transmits driving forces to move the grasping part, the grasping-portion wires running between the plurality of first guide pulleys and the grasping part without other pulleys, and a second rotating shaft (119) that turnably supports second guide pulleys located upstream of the first guide pulleys.

Patent Literatures 2 and 3 describe a medical treatment tool having an end portion such as a forceps. The forceps device comprises a grasping part, a support that holds the grasping part, bearings mounted in a hole extending through the support, a connection that is inserted in the bearing, a clevis that holds the connection, a plurality of grasping-portion wires that transmit driving forces to move the grasping part, a support wire that transmits a driving force to turn the support, first guide pulleys arranged coaxially with the connection, and a further pulley. The grasping-portion wires run over the first guide pulleys, and the support wire runs over the further pulley.

Patent Literature 4 describes an end tool of a surgical instrument. The end tool has jaws for performing a gripping operation, wires to move the jaws, an end tool hub, and a plurality of pulleys over which the wires for moving the jaws run. Some of the pulleys can perform a role of a type of ball bearing. Further, the end tool comprises a pitch pulley formed to be rotatable around a pin and fixedly connected to the end tool hub, and a pitch wire.

Patent Literature 5 describes a surgical tool and end effectors. A wrist assembly comprises jaws, cables, hinge cover, hinge-rotary body, a ball bearing set, and a hinge bearing race.

Patent Literature 6 describes a surgical instrument comprising a treatment tool comprising jaws, a wrist portion, and several wires. The surgical instrument includes bearingintegrated pulleys.

Patent Literature 7 describes a forceps device comprising a pair of grasping portions, a support that holds the pair of grasping portions, a first rotating shaft that turnably supports the support, a base member that holds the first rotating shaft, four guide pulleys arranged on the first rotating shaft, a second rotating shaft that turnably supports the pair of grasping portions and is held by the support, four jaw pulleys supported coaxially with the second rotating shaft, four wires running over the four guide pulleys and the four jaw pulleys, and wires for rotating the support about the first rotating shaft.

### Related Art List

### Patent Literature

Patent Literature 1: CN 107928792 A
Patent Literature 2: US 2018/311000 A1
Patent Literature 3: JP 2020 073013 A
Patent Literature 4: KR 102 308 214 B1
Patent Literature 5: US 2019/142531 A1
Patent Literature 6: US 2020/069382 A1
Patent Literature 7: JP 6 943522 B1

### Summary of the Invention

### Technical Problem

If the tensions of the grasping-portion wires are increased so as to increase the grasping force of the grasping part of the aforementioned forceps device, the frictional force between the first rotating shaft and the support increases, which lowers the controllability of the bending movement of the support including the grasping part about the first rotating shaft.

The present invention has been made in view of the aforementioned circumstances, and an exemplary object thereof is to provide a novel technology for improving the controllability of the operation of the forceps device.

### Solution to the problem

To solve the aforementioned problems, a forceps device according to claim 1 is provided. The forceps device according to an aspect of the present invention includes: a grasping part; a support that holds the grasping part; a shaft bearing mounted in a hole extending through the support; a first rotating shaft that is inserted in the bearing and turnably supports the support; a base member that holds the first rotating shaft; a plurality of grasping-portion wires that transmit driving forces to move the grasping part; a support wire that transmits a driving force to turn the support about the first rotating shaft; first guide pulleys arranged coaxially with the first rotating shaft, the grasping-portion wires running over the first guide pulleys; and a support pulley formed as part of the support coaxially with the first rotating shaft, the support wire running over the support pulley.

According to this aspect, the frictional force between the first rotating shaft and the support is reduced.

The bearing may be a rolling bearing. This further reduces the frictional force between the first rotating shaft and the support. Alternatively, the bearing may be a plain bearing.

The rolling bearing may have a diameter of 3.6 to 4.5 mm, and the first guide pulleys may have a diameter of 3.0 to 3.6 mm. The rolling bearing may have a diameter larger than that of the first guide pulleys.

### Advantageous Effects of Invention

According to the present invention, the controllability of the operation of the forceps device is improved.

### Brief Description of Drawings

FIG. 1 is a perspective view of a forceps device according to an embodiment.
FIG. 2 is a front view of the forceps device of FIG. 1 as viewed in a direction A.
FIG. 3 is a side view of the forceps device of FIG. 1 as viewed in a direction B.
FIG. 4 is a side view of the forceps device of FIG. 1 as viewed in a direction C.
FIG. 5 is a cross-sectional view of the forceps device illustrated in FIG. 3 along D-D.
FIG. 6 is a perspective view of a support according to the embodiment.
FIG. 7 is a front view of the support according to the embodiment.
FIG. 8 is a side view of the support according to the embodiment.
FIG. 9 is a perspective view illustrating a state in which a pair of jaw parts facing each other are to be fitted according to the embodiment.
FIG. 10 is a perspective view of the forceps device illustrated in FIG. 1 as viewed in a direction H1.
FIG. 11 is a perspective view of the forceps device illustrated in FIG. 1 as viewed in a direction H2.
FIG. 12 is a perspective view of the forceps device illustrated in FIG. 1 as viewed in a direction H3.
FIG. 13 is a perspective view of the forceps device illustrated in FIG. 1 as viewed in a direction opposite the direction H2.
FIG. 14 is a schematic view of the forceps device illustrated in FIG. 3 omitting a base member.
FIG. 15 is a schematic view of the forceps device illustrated in FIG. 4 omitting the base member.
FIG. 16 is a schematic view of the forceps device illustrated in FIG. 1 omitting wires.
FIG. 17 is a perspective view of the base member according to the embodiment.
FIG. 18 is a top view of the base member illustrated in FIG. 17 as viewed in a direction J1.
FIG. 19 is a front view of a stopper according to the embodiment.
FIG. 20 is a drawing for explaining the stopper positioned in the base member.

### Description of Embodiments

The present invention will now be described on the basis of an embodiment with reference to the drawings. Components, members, and processes that are the same as or equivalent to each other illustrated in the drawings are represented by the same reference numerals, and redundant explanation will not be repeated where appropriate. The embodiment is not to limit the invention, but is an example, and any feature or any combination of features described in the embodiment is not necessarily essential to the invention.

### [Forceps device]

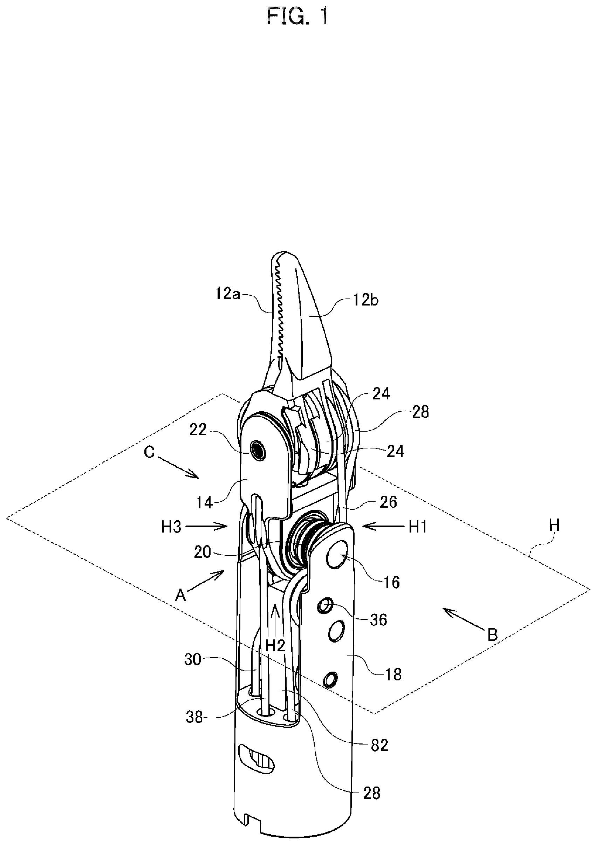

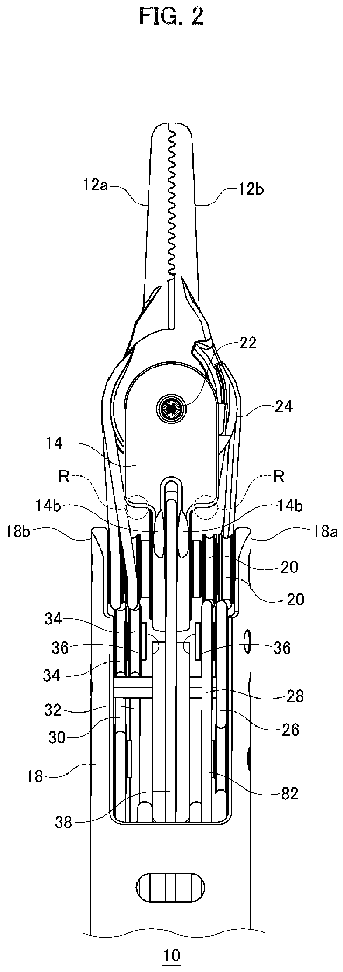

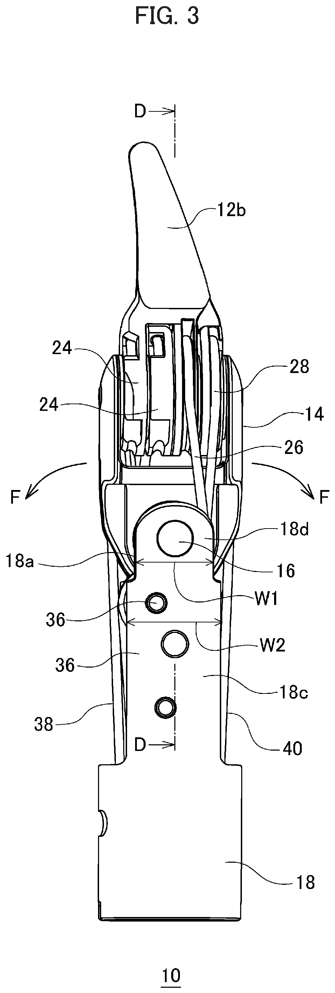

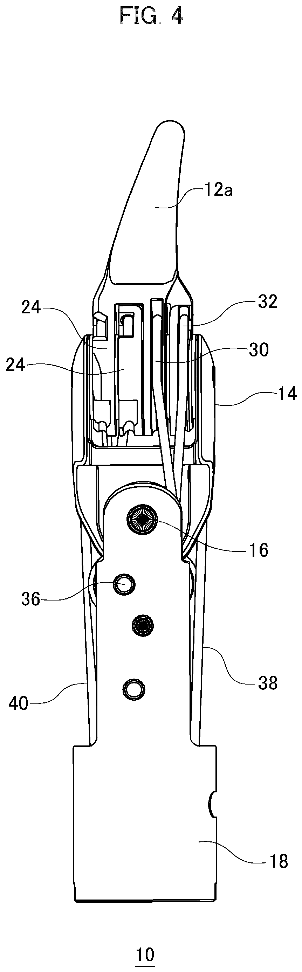

FIG. 1 is a perspective view of a forceps device according to an embodiment. FIG. 2 is a front view of the forceps device of FIG. 1 as viewed in a direction A. FIG. 3 is a side view of the forceps device of FIG. 1 as viewed in a direction B. FIG. 4 is a side view of the forceps device of FIG. 1 as viewed in a direction C.

The forceps device 10 illustrated in the drawings includes a pair of grasping portions 12a and 12b, a support 14 that holds the pair of grasping portions 12a and 12b, a first rotating shaft 16 that turnably supports the support 14, a base member 18 that holds the first rotating shaft 16, four guide pulleys 20 arranged coaxially with the first rotating shaft 16, a second rotating shaft 22 that turnably supports the pair of grasping portions 12a and 12b and is held by the support 14, four jaw pulleys 24 supported coaxially with the second rotating shaft 22, four wires 26, 28, 30 and 32 running over the four guide pulleys 20 and the four jaw pulleys 24, and wires 38 and 40 for turning the support 14 about the first rotating shaft 16.

### [Guide pulleys 20]

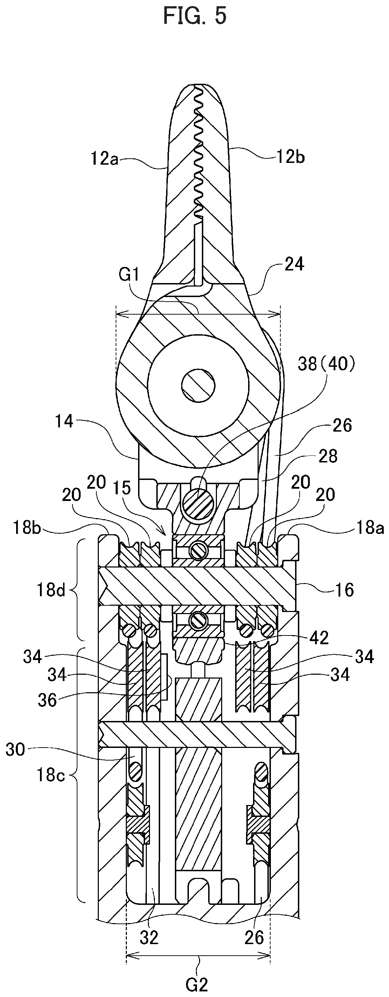

FIG. 5 is a cross-sectional view of the forceps device 10 illustrated in FIG. 3 along D-D. As illustrated in FIG. 5, the guide pulleys 20 are rotatably supported by the first rotating shaft 16 inserted in a bearing 15 mounted on the support 14. As illustrated in FIG. 5, the guide pulleys 20 according to the embodiment are arranged in such a manner that two guide pulleys 20 adjacent to each other are present on respective sides with respect to the support 14.

### [Base member]

Next, the base member 18 will be described. As illustrated in FIG. 2 and FIG. 5, the base member 18 according to the embodiment includes a pair of arms 18a and 18b that hold respective ends of the first rotating shaft 16, and third rotating shafts 36 that are held by the pair of arms 18a and 18b, respectively, and rotatably support four guide pulleys 34 located upstream of the guide pulleys 20. Note that a third rotating shaft 36 according to the embodiment is provided on each of the pair of arms 18a and 18b. In addition, the two rotating shafts 36 are arranged with their axial directions being parallel to each other but not being aligned with each other.

The arms 18a and 18b each have a base part 18c holding the third rotating shaft 36, and a distal end part 18d that holds the first rotating shaft 16 and that is thinner than the base part 18c. In other words, the distance between the distal end parts 18d is larger than the distance between the base parts 18c. Thus, as illustrated in FIG. 3, even when the wires 26 and 28 having the fleet angles are bent together with the support 14 around the first rotating shaft 16 (in the direction of an arrow F in FIG. 3), the wires 26 and 28 are less likely to interfere with the arms 18a and 18b.

Furthermore, the circumferential width W1 of the distal end part 18d of the arm 18a (the arm 18b) is smaller than the circumferential width W2 of the base part 18c thereof. Thus, a U-shaped recess is formed at the distal end part 18d at which interference with a wire needs to be addressed, and the circumferential width of the distal end part 18d is made larger than the circumferential width of the base part 18c, which minimizes deterioration of the stiffness of the arm.

In addition, as illustrated in FIG. 5, the jaw pulleys 24 according to the embodiment have an outer diameter G1, which is larger than the distance between the base parts 18c of the pair of arms 18a and 18b. Thus, in the forceps device 10 according to the embodiment, the jaw pulleys 24 having a large outer diameter relative to the inner diameter of the cylindrical base member 18 may be used.

### [Support]

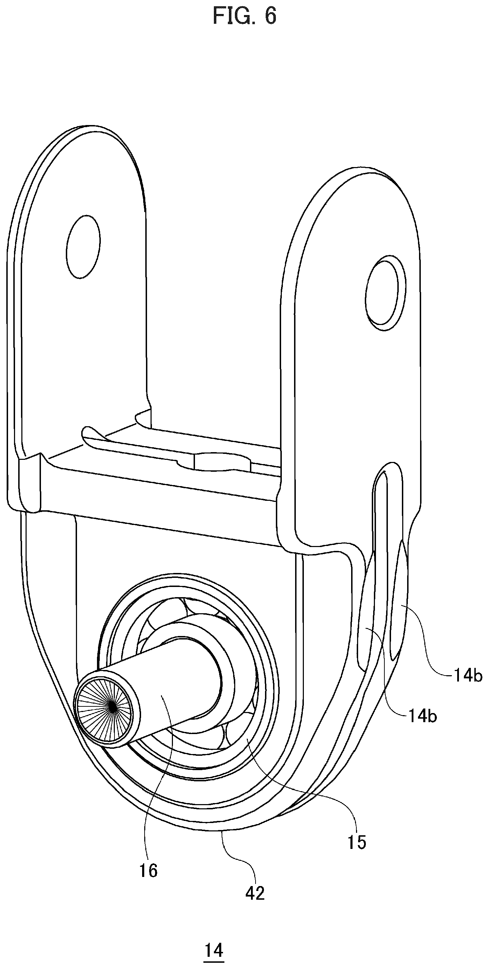

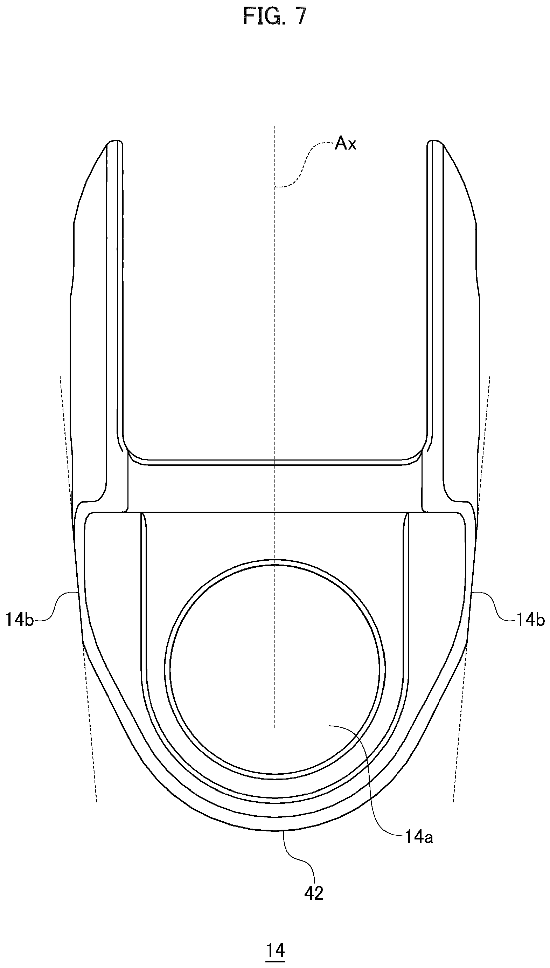

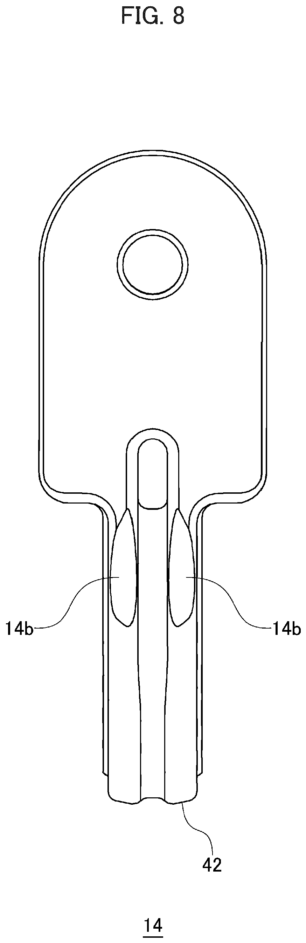

FIG. 6 is a perspective view of the support according to the embodiment. FIG. 7 is a front view of the support according to the embodiment. FIG. 8 is a side view of the support according to the embodiment. As illustrated in FIG. 5, the forceps device 10 according to the embodiment includes the pair of grasping portions 12a and 12b, the support 14 that holds the pair of grasping portions 12a and 12b, the first rotating shaft 16 that turnably supports the support 14, the base member 18 that holds the first rotating shaft 16, and a plurality of guide pulleys 20 arranged coaxially with the first rotating shaft 16.

As illustrated in FIG. 7, the support 14 has a circular through-hole 14a in which the bearing 15, which is a shaft bearing, is mounted. In addition, as illustrated in FIG. 6, the first rotating shaft 16, which rotatably supports the support 14, is inserted into the bearing 15 mounted on the support 14. The guide pulleys 20 are rotatably supported by the outer circumference of the first rotating shaft 16. Note that each rotating shaft is not limited to a shaft that rotates by itself, but may be any shaft that is the center of rotation of a member supported thereby, and may be a shaft fixed to another member.

In the forceps device 10 having such a structure, the support 14 in a state in which a plurality of guide pulleys 20 are supported by the outer circumference of the first rotating shaft 16 is held by the base member 18 with the first rotating shaft 16 therebetween. This configuration facilitates improvement in the easiness of assembly as compared with a case where the support 14 is held directly by the base member 18.

In addition, the base member 18 of the embodiment has the pair of arms 18a and 18b facing each other. The first rotating shaft 16 is firmly fixed in such a manner that the axial ends thereof are press-fitted to the pair of arms 18a and 18b. As a result, because the distal ends of the pair of arms 18a and 18b, which can be free ends, are fixed by the rotating shaft, the stiffness of the whole base member 18 increases.

Each of the wires 26, 28, 30 and 32 transmits a driving force to the grasping portion 12a or the grasping portion 12b to move the grasping portion 12a or the grasping portion 12b. Specifically, the wire 26 and the wire 32 run over the jaw pulleys 24 for the grasping portion 12b, and the grasping portion 12b moves in an opening direction when the wire 26 is pulled and moves in a closing direction when the wire 32 is pulled. The wire 28 and the wire 30 run over the jaw pulleys 24 for the grasping portion 12a, and the grasping portion 12a moves in a closing direction when the wire 28 is pulled and moves in an opening direction when the wire 30 is pulled.

In addition, each of the four guide pulleys 20 has a corresponding one of the wires 26, 28, 30 and 32 placed thereover. As illustrated in FIG. 2, each of the wires 26, 28, 30 and 32 passes between the corresponding ones of the guide pulleys 20 and guide pulleys 34 and runs over the corresponding one of the guide pulleys 20 from a lower side thereof (a side opposite the grasping portion 12a or 12b with respect to the first rotating shaft 16).

A support pulley 42, over which the wires 38 and 40 for transmitting a driving force for turning the support about the first rotating shaft 16 run, is formed integrally with part of the support 14. The support pulley 42 is arranged coaxially with the first rotating shaft 16. Thus, when one of the wires 38 and 40 is pulled to turn the support 14, the tension of the wire 38 or 40 running over the support pulley 42 presses the support 14 toward the first rotating shaft 16. A normal force received by the support 14 from the first rotating shaft 16 is thus generated, which contributes to an increase in frictional force. Alternatively, when one of the wires 26, 28, 30, and 32 is pulled to turn the grasping portions 12a and 12b, the tension of the wires 26, 28, 30, and 32 is applied to the second rotating shaft 22 via the jaw pulleys 24. The force applied to the inner circumference of the through-hole of the support 14 for supporting the second rotating shaft 22 is then transmitted to the circular through-hole 14a in which the bearing 15 is mounted, and the support 14 is thus pressed toward the first rotating shaft 16. In this case as well, a normal force received by the support 14 from the first rotating shaft 16 is generated, which contributes to an increase in frictional force.

In the forceps device 10 according to the embodiment, however, the bearing is provided between the support 14 and the first rotating shaft 16, which reduces the frictional force between the first rotating shaft 16 and the support 14. Note that, when the bearing 15 is a rolling bearing, the frictional force between the first rotating shaft 16 and the support 14 can further be reduced. Consequently, the movement of the grasping portions 12a and 12b becomes smoother, and the controllability of the forceps device 10 improves. Note that the bearing may be a plain bearing. The bearing 15 according to the embodiment may have a diameter of 3.6 to 4.5 mm, and the guide pulleys 20 may have a diameter of 3.0 to 3.6 mm. The bearing 15 may have a diameter larger than that of the guide pulleys 20.

### [Grasping portions]

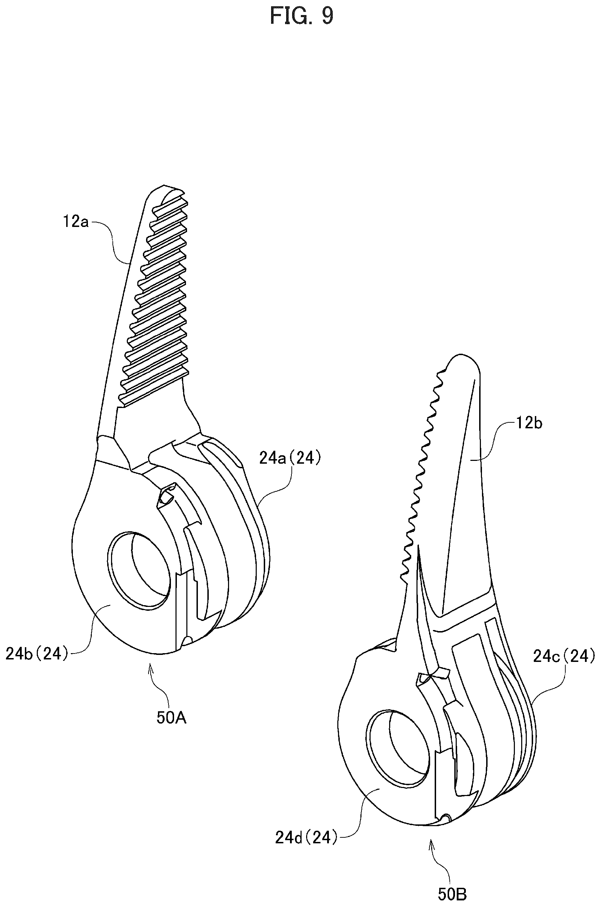

Next, jaw parts constituting the grasping portions according to the embodiment will be described in detail. FIG. 9 is a perspective view illustrating a state in which a pair of jaw parts facing each other are to be fitted according to the embodiment. Jaw parts 50A and 50B illustrated in FIG. 9 are parts having substantially the same shapes as each other. The jaw parts 50A and 50B have the grasping portion 12a and the grasping portion 12b, respectively, which move relative to each other to grasp an object. The grasping portion 12a is continuous with a jaw pulley 24a (first grasping portion pulley) and a jaw pulley 24b (second grasping portion pulley), which are formed in a bifurcated shape, and the grasping portion 12a and the jaw pulleys 24a and 24b integrally constitute the jaw part 50A. In addition, the grasping portion 12b is continuous with a jaw pulley 24c (third grasping portion pulley) and a jaw pulley 24d (fourth grasping portion pulley), which are formed in a bifurcated shape, and the grasping portion 12b and the jaw pulleys 24c and 24d integrally constitute the jaw part 50B.

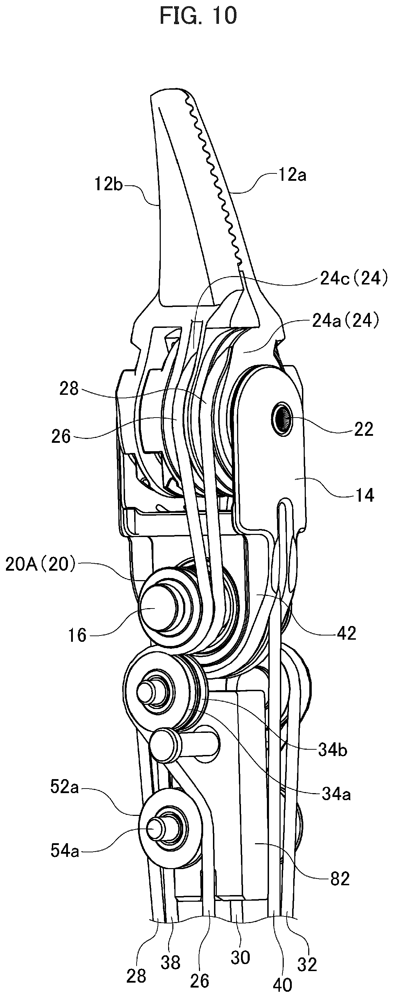

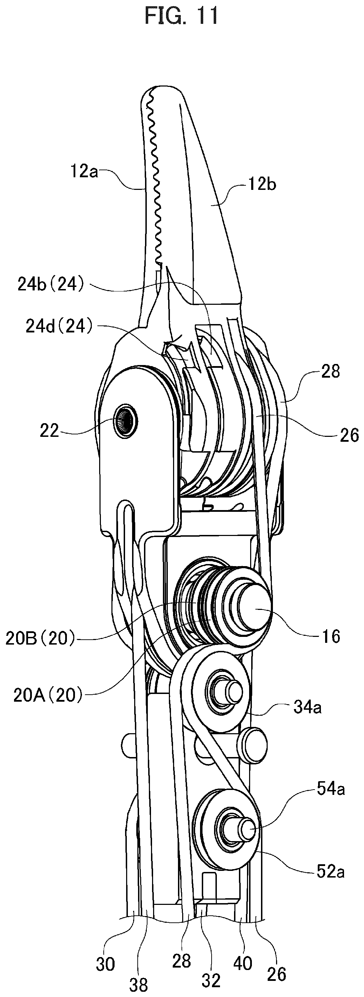

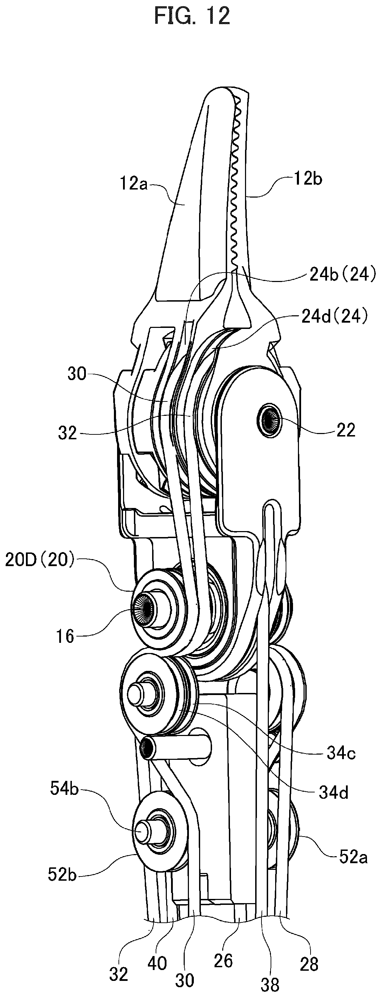

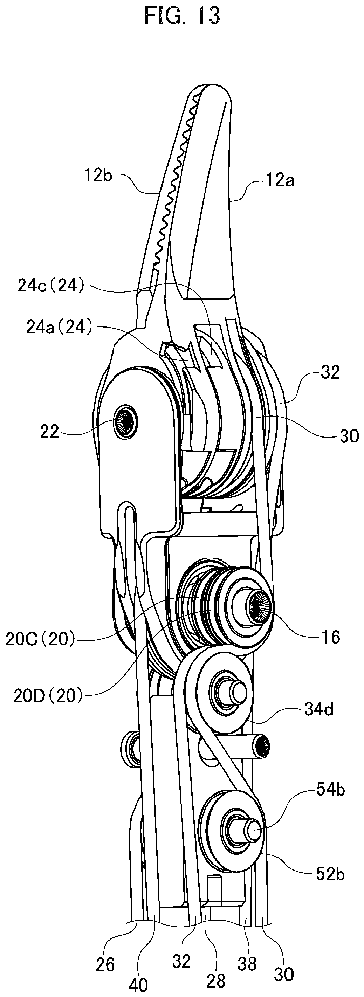

FIG. 10 is a perspective view of the forceps device 10 illustrated in FIG. 1 as viewed in a direction H1. FIG. 11 is a perspective view of the forceps device 10 illustrated in FIG. 1 as viewed in a direction H2. FIG. 12 is a perspective view of the forceps device 10 illustrated in FIG. 1 as viewed in a direction H3. FIG. 13 is a perspective view of the forceps device 10 illustrated in FIG. 1 as viewed in a direction opposite the direction H2. Note that the directions H1 to H3 are directions within a horizontal plane H including the first rotating shaft 16. In addition, the base member 18 is not illustrated in FIGS. 10 to 13.

As illustrated in FIGS. 10 to 13, the jaw pulley 24a, the jaw pulley 24c, the jaw pulley 24b, and the jaw pulley 24d are rotatably supported in this order by the second rotating shaft 22. In addition, the guide pulley 20A, the guide pulley 20B, the guide pulley 20C, and the guide pulley 20D are rotatably supported in this order by the first rotating shaft 16. The wires 26, 28, 30 and 32, which are grasping-portion wires for transmitting driving forces for causing the grasping portions 12a and 12b to perform opening and closing movements, run between the guide pulleys 20A to 20D and the grasping portions 12a and 12b without other pulleys. Note that the driving forces are input from an actuator unit outside of the forceps device 10. This configuration can shorten the distance between the first rotating shaft 16 and the second rotating shaft 22 as illustrated in FIG. 10, etc., and can therefore increase the torque (operation force) of the grasping portions 12a and 12b. Consequently, the forceps device 10 with high controllability of the grasping portions 12a and 12b can be provided.

### [How wires run over pulleys]

Next, the manner in which the wires 26, 28, 30 and 32 according to the embodiment run over the pulleys will be described in detail. The wire 26 runs between the guide pulley 20A and the jaw pulley 24c. The wire 28 runs between the guide pulley 20B and the jaw pulley 24a. The wire 30 runs between the guide pulley 20D and the jaw pulley 24b. The wire 32 runs between the guide pulley 20C and the jaw pulley 24d.

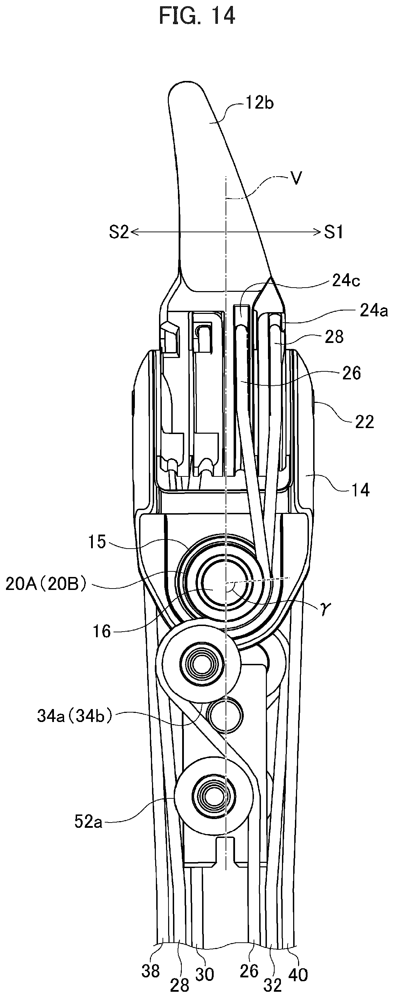

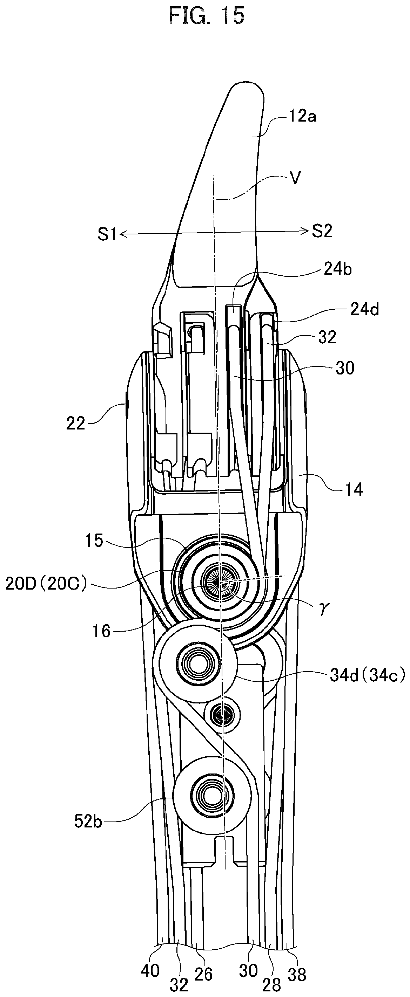

FIG. 14 is a schematic view of the forceps device 10 illustrated in FIG. 3 omitting base member 18. FIG. 15 is a schematic view of the forceps device 10 illustrated in FIG. 4 omitting the base member.

As illustrated in FIGS. 10, 11, and 14, the wire 26 is passed over a first side S1 of the guide pulley 20A with respect to a vertical cross section V including the first rotating shaft 16 and being perpendicular to the second rotating shaft 22, and then fixed to the jaw pulley 24c located on the first side S1 with respect to the vertical cross section V. The wire 28 is passed over the first side S1 of the guide pulley 20B with respect to the vertical cross section V, and then fixed to the jaw pulley 24a located on the first side S1 with respect to the vertical cross section V.

In addition, as illustrated in FIGS. 12, 13, and 15, the wire 30 is passed over the second side S2 of the guide pulley 20D with respect to the vertical cross section V, and then fixed to the jaw pulley 24b located on the second side with respect to the vertical cross section V. The wire 32 is passed over the second side S2 of the guide pulley 20C with respect to the vertical cross section V, and then fixed to the jaw pulley 24d located on the second side S2 with respect to the vertical cross section V. As a result, the four grasping-portion wires are fixed to the four grasping portion pulleys, respectively, without intersecting with each other.

Furthermore, the forceps device 10 includes the guide pulley 34a on the upstream side of the guide pulley 20A (on the side opposite the grasping portions) and on the second side S2 with respect to the vertical cross section V, the guide pulley 34b on the upstream side of the guide pulley 20B and on the second side S2 with respect to the vertical cross section V, the guide pulley 34c on the upstream side of the guide pulley 20C and on the first side S1 with respect to the vertical cross section V, and the guide pulley 34d on the upstream side of the guide pulley 20D and on the first side S1 with respect to the vertical cross section V.

As a result, when the support 14 is bent in either direction about the first rotating shaft 16, one or more of the wires 26, 28, 30 and 32 come in contact with the associated one or more of the guide pulleys 20A to 20D, which stabilizes the controllability when the support 14 is turned. More specifically, when the support 14 is bent from the state illustrated in FIG. 14 toward the second side S2, the wires 26 and 28 come in contact with the guide pulleys 20A and 20B, respectively. In addition, when the support 14 is bent from the state illustrated in FIG. 15 toward the first side S1, the wires 30 and 32 come in contact with the guide pulleys 20C and 20D, respectively. Thus, in either case, such a state in which none of the wires are in contact with the guide pulleys 20A to 20D (run over the guide pulleys 20A to 20D) is avoided. As a result, the controllability when the support 14 is bent about the first rotating shaft 16 is stabilized.

### [Sealing of base member]

Because the grasping portions 12a and 12b of the forceps device 10 according to the embodiment are operated inside the abdominal cavity of a patient, it is necessary to devise a way to prevent gas around the grasping portions 12a and 12b from leaking out through the forceps device 10 so that the air pressure in the abdominal cavity, which is increased to be higher than the atmospheric pressure, does not lower. In the present embodiment, sealing is therefore provided in a region including the base member 18.

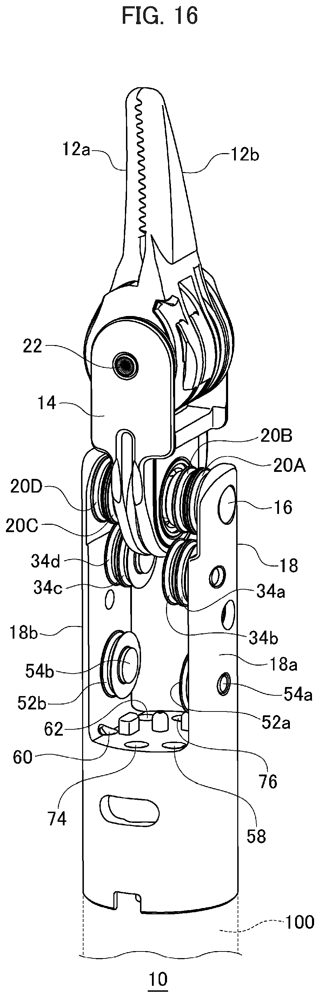

FIG. 16 is a schematic view of the forceps device illustrated in FIG. 1 omitting wires. As illustrated in FIG. 16, the forceps device 10 includes the pair of grasping portions 12a and 12b, the support 14 that holds the pair of grasping portions 12a and 12b, the first rotating shaft 16 that turnably supports the support 14, the base member 18 that holds the first rotating shaft 16, a plurality of wires 26, 28, 30 and 32 (not illustrated in FIG. 16; see FIG. 2) for transmitting driving forces to the pair of grasping portions 12a and 12b to move the grasping portions 12a and 12b, a guide pulley 52a for guiding the wire 26, a guide pulley 52b for guiding the wire 30, and support shafts 54a and 54b that rotatably support the guide pulleys 52a and 52b. The base member 18 is located between the grasping portions 12a and 12b and a shaft 100 of the forceps device 10.

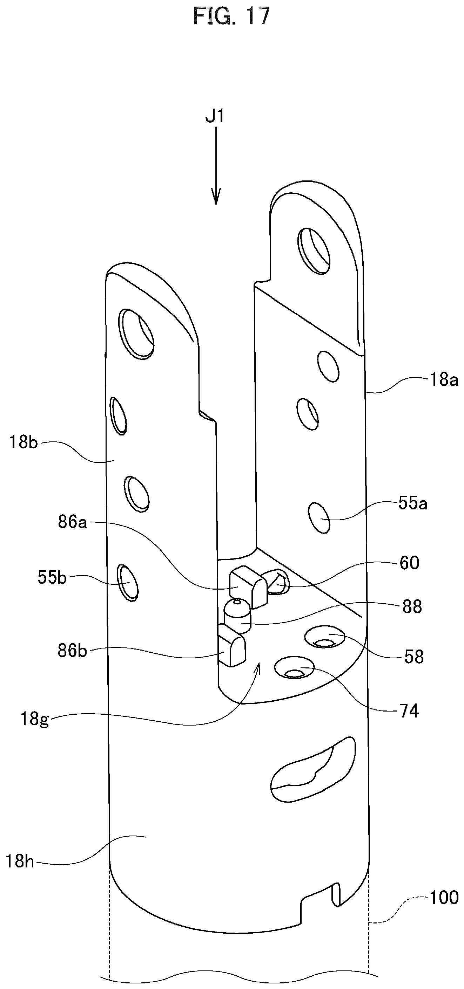

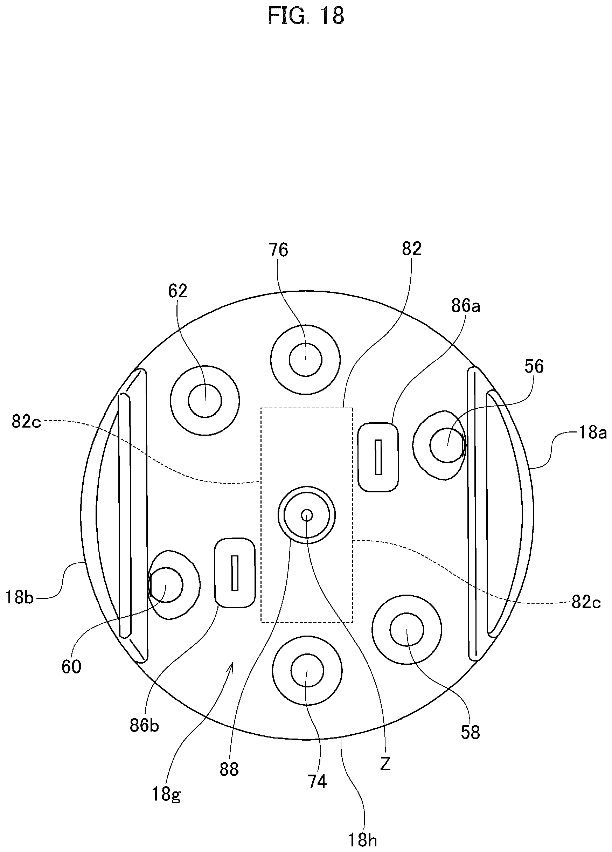

FIG. 17 is a perspective view of the base member according to the embodiment. FIG. 18 is a top view of the base member illustrated in FIG. 17 as viewed in a direction J1.

The base member 18 includes a partition part 18g having a plurality of grasping-portion-wire holes 56, 58, 60 and 62 through which the wires 26, 28, 30 and 32, respectively, pass, and the pair of arms 18a and 18b which extend from an outer edge of the partition part 18g toward the grasping part (upward in FIG. 17) and to which the support shafts 54a and 54b are fixed. The arms 18a and 18b have fitting holes 55a and 55b, respectively, into which ends of the support shafts 54a and 54b that rotatably support the pair of guide pulleys 52a and 52b are fitted.

The partition part 18g is a partition formed between a cylindrical part 18h and the pair of arms 18a and 18b. The grasping-portion-wire holes 56 and 58 are a pair of grasping-portion-wire holes that are adjacent to each other at the closet distance, and the grasping-portion-wire holes 60 and 62 are a pair of grasping-portion-wire holes that are adjacent to each other at the closest distance.

As illustrated in FIG. 10, the wires 26 and 28 in a state being guided by the guide pulleys 34a and 34b are arranged together. Thus, for passing the wires 26 and 28 in this state through the partition part 18g, a large hole through which two wires can pass needs to be formed through the partition part 18g or two small holes through each of which one wire can pass need to be formed through the partition part 18g. In the case of a large hole, the airtightness of sealing with sealing resin, which will be described later, may be lowered. In contrast, in the case of two adjacent small holes, the wall between the holes becomes thin, and the strength of an area of the base member 18 where the holes are formed may therefore be lowered.

Hence, in the forceps device 10 according to the embodiment, the wire 26 is guided by the guide pulley 52a, so that the wire 26 is separated from the wire 28. Similarly, the wire 30 is guided by the guide pulley 52b, so that the wire 30 is separated from the wire 32. In this manner, the grasping-portion-wire hole 56 through which the wire 26 passes and the grasping-portion-wire hole 58 through which the wire 28 passes can be separated from each other. Similarly, the grasping-portion-wire hole 60 through which the wire 30 passes and the grasping-portion-wire hole 62 through which the wire 32 passes can be separated from each other.

In addition, the base member 18 includes an elastic sealing member (having a type A durometer hardness of 30 to 70) arranged on one side (on one side opposite the side in which the grasping portions 12a and 12b are located) with respect to the partition part 18g. The sealing member is a silicone resin film having a thickness of about 0.5 to 2 mm, for example.

The sealing member has a plurality of sealing holes at positions corresponding to those of the grasping-portion-wire holes 56, 58, 60 and 62. This enables sealing with the sealing member, with the wires 26, 28, 30 and 32 passing through the partition part 18g of the base member 18. The wires have a diameter φ of 0.4 to 0.5 mm, and the diameter of the grasping-portion-wire holes is preferably slightly larger than the wire diameter.

The sealing holes have a diameter that is smaller than the diameter of the grasping-portion-wire holes and smaller than the diameter (wire diameter φ) of the grasping-portion wires. As a result, even when a gap is present between a grasping-portion-wire hole and a grasping-portion wire, the sealing hole and the grasping-portion wire are in close contact with each other, which reduces leakage of gas from the grasping part side to the outside of the forceps device 10 via the base member 18.

As illustrated in the drawings, the four grasping-portion-wire holes according to the embodiment are formed through the partition part 18g at certain distances from each other. Thus, the sealing holes formed at positions corresponding to those of the grasping-portion-wire holes are also separated from the adjacent sealing holes.

In addition, the forceps device 10 according to the embodiment includes the pair of wires 38 and 40 for turning the support 14 about the first rotating shaft 16 in addition to the wires 26, 28, 30 and 32, which are grasping-portion wires. The partition part 18g of the base member 18 therefore has support-wire holes 74 and 76 through which the wires 38 and 40, respectively, pass. The support-wire holes 74 and 76 are formed at point-symmetric positions with respect to the center Z of the partition part 18g (the central axis of the cylindrical part 18h).

Furthermore, the sealing member has a plurality of sealing holes formed at positions corresponding to those of the support-wire holes 74 and 76. This enables sealing with the sealing member, with the wires 38 and 40 passing through the partition part 18g of the base member 18. The diameter of the support-wire holes is preferably slightly larger than the wire diameter. Furthermore, the arrangement of the pulleys of the forceps device 10 according to the embodiment is devised, so that a plurality of holes for wires formed in the partition part 18g of the base member 18 can be suitably separated from each other. This reduces such a problem as unintended integration of holes for wires in forming the sealing member having a plurality of holes for wires.

### [Turning restricting mechanism]

In the forceps device 10 illustrated in FIG. 2, in a state in which the driving force for turning the support 14 is transmitted via the wires 38 and 40, the grasping portions 12a and 12b are bent at an angle in a control range (±80° in the embodiment) that prevents part of the support 14 and the grasping-portion wires 26, 28, 30, and 32 from interfering with each other. In a state in which the forceps device 10 is detached from a power source or a main unit of a robot, however, the support 14 is freely turnable beyond the angles in the control range, and a partial region R of the support illustrated in FIG. 2 may therefore touch the grasping-portion wires 26, 28, 30, and 32.

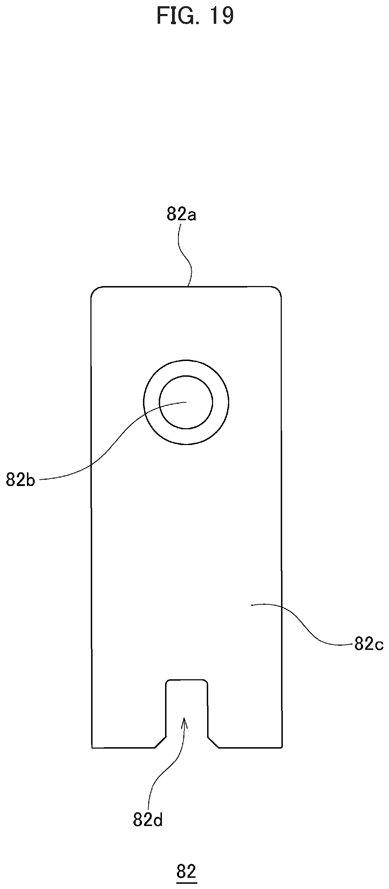

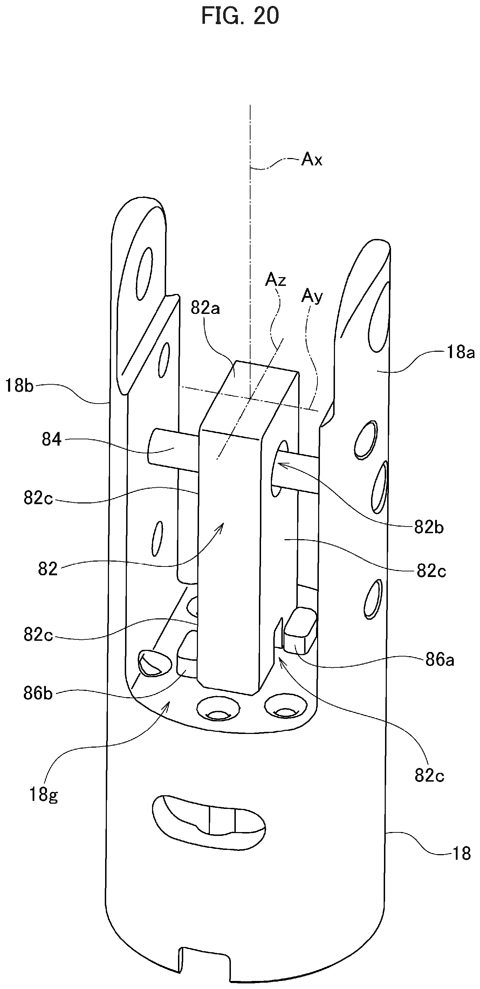

Thus, the forceps device 10 according to the embodiment includes a turning restricting mechanism for restricting turning of the support to prevent the turning support 14 from touching the grasping-portion wires 26, 28, 30, and 32. The turning restricting mechanism includes a stopper having a face with which part of the turning support comes into contact. FIG. 19 is a front view of the stopper according to the embodiment. FIG. 20 is a drawing for explaining the stopper positioned in the base member 18.

The stopper 82 is a block-like rectangular parallelepiped member having a flat face 82a, which faces the support 14, serving as the face with which the turning support comes into contact. When a contact face 14b of the support 14 comes into contact with the flat face 82a (see FIGS. 2 and 7 to 9), further turning of the support 14 is restricted. In this manner, the turning restricting mechanism is achieved with a simple part. Furthermore, because the support 14 is prevented from touching the grasping-portion wires 26, 28, 30, and 32, the durability of the grasping-portion wires is improved, and therefore the product life of the forceps device 10 is extended.

As described above, because the support 14 and the stopper 82 directly come in contact with each other, the turning restricting mechanism according to the embodiment can more reliably prevent contact between the support and the grasping-portion wires.

Furthermore, as illustrated in FIG. 7, the contact face 14b formed at an outer edge of the support 14 is a flat surface inclined with respect to the central axis Ax (the axis passing the center Z shown in FIG. 18) of the base member 18 at the neutral position (the state illustrated in FIG. 3, for example) at which the support 14 is not turned to the left or to the right. The support 14 is designed to come into contact with the stopper 82 when the turning angle from the neutral position becomes a contact angle β (α<β) beyond the angles α in the predetermined control range. This prevents the support 14 from turning at angles larger than the contact angle β while permitting turning at the angles α in the predetermined control range, which prevents hindrance to turning control of the support 14 by the stopper 82.

Note that the contact angle β is preferably 70° or larger, and may be larger than 90°. This enables control of the turning of the support at least up to 70°. If the contact angle β is too large, however, the support 14 may touch the grasping-portion wires before the support 14 comes into contact with the stopper 82. The contact angle β is therefore smaller than a turning angle at which the support 14 touches a grasping-portion wire. Specifically, the contact angle β may be smaller than 110°, or may be smaller than 100°.

Note that, if the contact angle β varies, the support 14 may unintentionally touch a grasping-portion wire. Positioning of the stopper 82 is therefore important. The stopper 82 according to the embodiment is positioned relative to the base member 18. As a result, both the support 14 and the stopper 82 are at predetermined positions relative to the base member 18, which improves the accuracy of relative positions of the support 14 and the stopper 82.

Specific positioning in the embodiment will be explained. As illustrated in FIG. 19, a through-hole 82b is formed in a side face of the stopper 82. In addition, as illustrated in FIG. 20, the pair of arms 18a and 18b of the base member 18 holds a positioning pin 84 that is inserted in and extending through the through-hole 82b of the stopper 82. Thus, the stopper 82 can be accurately positioned in the direction of the central axis Ax of the base member 18. Furthermore, because the pair of arms 18a and 18b holds the positioning pin 84, the strength of the arms is increased.

In addition, the partition part 18g at the base part of the arms 18a and 18b of the base member 18 has a plurality of projections 86a, 86b, and 88 used for positioning of the stopper 82. The shapes and the arrangement of the projections 86a and 86b are set for positioning in a direction Ay intersecting the central axis Ax when the projections 86a and 86b come in contact with a pair of side faces 82c, which are opposite each other, of the stopper 82. In addition, the shape and the arrangement of the projection 88 are set for positioning in a direction Az intersecting the central axis Ax and the direction Ay when the projection 88 is fitted into a groove 82d formed at a lower portion of the stopper 82. Thus, the stopper 82 can be accurately positioned in the directions Ay and Az intersecting the axial direction Ax of the base member 18.

In addition, as illustrated in FIGS. 10 and 18, the stopper 82 is arranged in a region surrounded by a plurality of grasping-portion wires and support wires. Thus, the region surrounded by the plurality of wires is occupied by the stopper 82, which prevents foreign substances from staying inside the forceps device 10 (in a region surrounded by the wires) as compared with a case where no stopper is provided.

### [Arrangement of pulleys]

In the forceps device 10 according to the embodiment, the arrangement of the pulleys is devised so as to improve the durability of the wires while allowing some degree of wire winding angle. Specifically, as illustrated in FIG. 14, the guide pulleys 34a and 34b are shifted toward one side (toward S2) relative to immediately below the guide pulleys 20A and 20B when the first rotating shaft 16 is viewed in front in the axial direction in a state in which the leading ends of the grasping portions 12a and 12b point upward. Furthermore, the guide pulleys 34a and 34b are arranged so that the winding angle γ of the grasping-portion wires 26 and 28 around the guide pulleys 20A and 20B is 65° or larger at the neutral position at which the support 14 is not turned to the left or to the right.

This enables comfortable operation control with the turning angle of the support 14, which holds the grasping portions 12a and 12b, being at least within a range of ±65°. Note that the winding angle γ may be 89° or larger. This enables comfortable operation control with the turning angle of the support, which holds the grasping portions 12a and 12b, being at least within a range of ±89°.

The guide pulley 52a is located upstream of the guide pulleys 34a and 34b, and the grasping-portion wire 26 runs over the guide pulley 52a. The guide pulley 52a is shifted toward one side (toward S2) relative to immediately below the guide pulleys 20A and 20B and shifted toward the other side (toward S1) relative to immediately below the guide pulleys 34a and 34b when the first rotating shaft 16 is viewed in front in the axial direction in the state in which the leading ends of the grasping portions 12a and 12b point upward. This increases the radius of curvature of an S-shaped curve of the grasping-portion wire 26 running over the guide pulley 34a and the guide pulley 52a. Furthermore, as a result of the increase in the radius of curvature of the S-shaped curve of the grasping-portion wire 26, bending stress generated in the grasping-portion wire 26 is lowered, and the durability of the grasping-portion wires relating to opening and closing movements of the grasping portions 12a and 12b is improved.

Similarly, as illustrated in FIG. 15, the guide pulleys 34d and 34c is shifted toward one side (toward S1) relative to immediately below the guide pulleys 20D and 20C when the first rotating shaft 16 is viewed in front in the axial direction in the state in which the leading ends of the grasping portions 12a and 12b point upward. In addition, the guide pulleys 34d and 34c are arranged so that the winding angle γ of the grasping-portion wires 30 and 32 around the guide pulleys 20D and 20C is 65° or larger at the neutral position at which the support 14 is not turned to the left or to the right.

The guide pulley 52b is located upstream of the guide pulleys 34d and 34c, and the grasping-portion wire 30 runs over the guide pulley 52b. The guide pulley 52b is shifted toward one side (toward S1) relative to immediately below the guide pulleys 20D and 20C and shifted toward the other side (toward S2) relative to immediately below the guide pulleys 34d and 34c when the first rotating shaft 16 is viewed in front in the axial direction in the state in which the leading ends of the grasping portions 12a and 12b point upward. This increases the radius of curvature of an S-shaped curve of the grasping-portion wire 30 running over the guide pulley 34d and the guide pulley 52b. Furthermore, as a result of the increase in the radius of curvature of the S-shaped curve of the grasping-portion wire 30, bending stress generated in the grasping-portion wire 30 is lowered, and the durability of the grasping-portion wires relating to opening and closing movements of the grasping portions 12a and 12b is improved.

Note that, as illustrated in FIG. 16, the guide pulleys 52a and 52b are located on respective sides of the inner wall of the base member 18. The guide pulleys 52a and 52b are also rotatably supported by different support shafts 54a and 54b, respectively, which are held by the base member 18. This enables the guide pulley 52a and the guide pulleys 34a and 34b to be arranged on the same side (left side in the embodiment) when the guide pulley 20A is viewed from the front. Similarly, the guide pulley 52b and the guide pulleys 34c and 34d can be arranged on the same side (left side in the embodiment) when the guide pulley 20D is viewed from the front.

Note that the base member 18 according to the embodiment is a cylindrical part having an outer diameter of 7 to 9 mm. Furthermore, the guide pulleys 20A to 20D have a diameter of 3.0 to 3.6 mm, the guide pulleys 34a to 34d have a diameter of 3.0 to 3.6 mm, and the guide pulleys 52a and 52b have a diameter of 3.0 to 3.6 mm. As a result, in a forceps device having a very small outer diameter, the radius of curvature of each S-shaped curve can be increased while appropriate winding angles are achieved.

### Industrial Applicability

The present invention can be used for a manipulator of a surgical robot.

### Reference Signs List

- 10: forceps device
- 12a: grasping portion
- 12b: grasping portion
- 14: support
- 14a: through-hole
- 14b: contact face
- 15: bearing
- 16: first rotating shaft
- 18: base member
- 18a: arm
- 18b: arm
- 18g: partition part
- 20: guide pulley
- 22: second rotating shaft
- 24: jaw pulley
- 26, 28, 30, 32: grasping-portion wire
- 34: guide pulley
- 36: third rotating shaft
- 52a, 52b: guide pulley
- 82: stopper
- 82a: flat face
- 82b: through-hole
- 82c: side face
- 82d: groove
- 84: positioning pin
- 86a, 86b: projection
- 88: projection

## Claims

1. A forceps device (10) comprising:
a grasping part;
a support (14) that holds the grasping part and has a circular through-hole (14a);
a bearing (15) which is a shaft bearing and mounted in the circular through-hole (14a) extending through the support (14);
a first rotating shaft (16) that is inserted in the bearing (15) and turnably supports the support (16);
a base member (18) that holds the first rotating shaft (16) ;
a plurality of grasping-portion wires (26, 28, 30, 32) that transmit driving forces to move the grasping part;
a support wire (38, 40) that transmits a driving force to turn the support (14) about the first rotating shaft (16) ;
first guide pulleys (20) arranged coaxially with the first rotating shaft (16) and rotatably supported by an outer circumference of the first rotating shaft (16), the grasping-portion wires (26, 28, 30, 32) running over the first guide pulleys(20);
a support pulley (42) formed as part of the support (14) coaxially with the first rotating shaft (16), the support wire (38, 40) running over the support pulley (42).

2. The forceps device (10) according to claim 1, wherein
the bearing (15) is a rolling bearing.

3. The forceps device (10) according to claim 2, wherein
the rolling bearing has a diameter of 3.6 to 4.5 mm, and
the first guide pulleys (20) have a diameter of 3.0 to 3.6 mm.

## Patentansprüche

1. Zangenvorrichtung (10), umfassend:
einen Greifteil;
eine Halterung (14), die den Greifteil hält und ein kreisförmiges Durchgangsloch (14a) aufweist;
ein Lager (15), das ein Wellenlager ist und im kreisförmigen Durchgangsloch (14a) (14) montiert ist, das sich durch die Halterung (14) erstreckt;
eine erste Rotationswelle (16), die im Lager (15) eingesetzt ist und die Halterung (14) drehbar hält;
ein Basiselement (18), das die erste Rotationswelle hält (16);
eine Vielzahl von Greifabschnittsdrähten (26, 28, 30, 32), die Treibkräfte übertragen, um den Greifteil zu bewegen;
ein Halterungsdraht (38, 40), der eine Treibkraft überträgt, um die Halterung (14) um die erste Rotationswelle (16) zu drehen;
erste Führungsrollen (20), die mit der ersten Rotationswelle (16) koaxial angeordnet sind und von einem Außenumfang der ersten Rotationswelle (16) drehbar gehalten werden, wobei die Greifabschnittsdrähte (26, 28, 30, 32) über die ersten Führungsrollen laufen (20);
eine Halterungsrolle (42), die als Teil der Halterung (14) koaxial mit der ersten Rotationswelle (16) gebildet ist, wobei der Halterungsdraht (38, 40) über die Halterungsrolle (42) läuft.

2. Zangenvorrichtung (10) gemäß Anspruch 1, wobei
das Lager (15) ein Wälzlager ist.

3. Zangenvorrichtung (10) gemäß Anspruch 2, wobei
das Wälzlager einen Durchmesser von 3,6 bis 4,5 mm aufweist, und
die ersten Führungsrollen (20) einen Durchmesser von 3,0 bis 3,6 mm aufweisen.

## Revendications

1. Un dispositif de forceps (10) comprenant :
une partie de préhension ;
un support (14) qui maintient la partie de préhension et possède un trou circulaire (14a) ;
un palier (15) qui est un palier d'arbre monté dans le trou circulaire (14a) qui s'étend à travers le support (14) ;
un premier arbre rotatif (16) qui est inséré dans le palier (15) et qui soutient tournablement le support (14) ;
un élément de base (18) qui maintient le premier arbre rotatif (16) ;
une pluralité de fils de la partie de préhension (26, 28, 30, 32) qui transmettent des forces motrices pour déplacer la partie de préhension ;
un fil de support (38, 40) qui transmet une force motrice pour faire tourner le support (14) autour du premier arbre rotatif (16) ;
premières poulies de guidage (20) disposées coaxialement avec le premier arbre rotatif (16) et soutenues de façon rotative par une circonférence extérieure du premier arbre rotatif (16), les fils de la partie de préhension (26, 28, 30, 32) passant sur les premières poulies de guidage (20) ;
une poulie de support (42) formée en tant que partie du support (14) de façon coaxiale avec le premier arbre rotatif (16), le fil de support (38, 40) passant sur la poulie de support (42).

2. Le dispositif de forceps (10) selon la revendication 1, dans lequel
le palier (15) est un palier à rouleau.

3. Le dispositif de forceps (10) selon la revendication 2, dans lequel
le palier à rouleau a un diamètre de 3,6 à 4,5 mm, et
les premières poulies de guidage (20) ont un diamètre de 3,0 à 3,6 mm.
